# EUROPEAN PATENT APPLICATION

(11) **EP 3 622 987 A1**
(43) Date of publication of application: **18.03.2020**
(21) Application number: 18193761.6
(22) Date of filing: 11.09.2018
(51) Int. Cl.: A61M 5/31, A61M 5/315

(54) **A METHOD FOR ASSEMBLY OF AN INJECTION OR INFUSION DEVICE**

(71) Applicant: Tecpharma Licensing AG, 3401 Burgdorf (CH)
(72) Inventor: Bigler, Bernhard, 3362 Niederönz (CH); Pirker, Gerhard, 5600 Lenzburg (CH)

(57) **Abstract**

An assembly method for an injection or infusion device comprising: Providing a first housing part including a cartridge closed by a plunger. Providing a second housing part connectable to the first housing part comprising: a drive mechanism for advancing a piston rod, a dose setting mechanism and a dose limiting mechanism configured to count and accumulate the set dose units and limit dose setting. Measuring the position of the cartridge plunger and the position of the piston rod and calculating a distance (d) between the piston rod and the plunger if the first and second housing parts were connected with each other. Selecting a dose and advance the piston rod using the dose setting and drive mechanism if the calculated distance (d) is between a minimum distance and a maximum distance thereby operating the dose limiting mechanism. Connecting the first housing part comprising the cartridge and the second housing part to form the injection or infusion device.

## Description

### Specification

The invention relates to an assembly of components for an injection or an infusion device that allows a user to inject a single preset dose (a bolus injector) or allows a user to select single or multiple doses of a liquid medicament and dispense the selected doses into a patient, preferably by injecting the selected dose. In particular, the present invention relates to a method for assembly of such an injection or infusion device.

### Background

Drug delivery devices such as injection or infusion devices that are used to deliver a medicament to the patient are well known in the prior art. Such injection or infusion devices fulfill essentially the same purpose as a syringe, e.g. injection of a liquid medicament into a patient, preferably by subcutaneous delivery through a needle that is attached or attachable to the injection or infusion device. These injection or infusion devices have to meet a number of specific requirements, as many users apply the injection themselves without the help of a health care professional, or users may be visually or physically impaired. Therefore these injection or infusion devices must be robust, easy to use in terms of setting and delivery of the medicament and safe in operation, for example preventing setting of a dose that cannot be delivered anymore form the device or prevent under- or over- dosing. Such devices must be inexpensive, especially when such devices are single-use devices. Also the manufacturing methods for producing such devices are required to allow for cost effective production of, and particularly prevent advert use of the assembled device.

The medicament is preferably delivered from a containment holding the fluid. Examples of such a containment are a cartridge, an ampoule or a prefilled syringe. A cartridge comprises a barrel forming a hollow cylinder which is closed on one side by a pierceable septum and on the opposite side by a plunger. The medicament is sealed between the septum and the plunger, and once the septum is pierced by a hollow needle, advancement of the plunger by a delivery mechanism will expel medicament through the needle. The barrel is preferably made of glass having excellent barrier properties and virtually no leachables that can diffuse into the liquid medicament. A disadvantage of glass is that the dimensional tolerances are relatively high compared to plastic (COC) containers. The glass barrel has an opening for receiving the plunger and a neck region for attaching the septum, also the position and shape of the neck portion is prone to dimensional tolerances that are inherently linked to the glass manufacturing process. Such cartridges can be filled with medicament in two different ways, either the septum is first attached to the neck portion of the barrel and subsequently the medicament is filled into the opening and finally the opening is closed with the plunger using a positioning tube. Alternatively, the plunger is first positioned into the opening of the barrel, the medicament is filled from the neck portion and finally the septum is attached to the neck using a crimp. Depending on the method used, the axial position of the plunger in the glass barrel can be subjected to a tolerance field of several millimeters.

The containment for the medicament is attached to a housing of dose setting and delivery mechanism using a containment holder, preferably a cartridge holder. The containment holder can be reversibly attached to the housing using a bayonet or screw type of connection for a reusable device and a snap-fit, adhesive of welded connection for a disposable device. The housing and the containment holder have suitable arresters or snap fit connectors for connecting the two parts. The containment holder and the housing with the specific connectors have certain dimensional tolerances and, in case plastic parts are used, ageing of the material can change the dimensions of the parts due to shrinkage.

The dose setting and delivery mechanism comprises a dose setting element, for example a dosing sleeve that can be axially and/or rotationally displaced with respect to the housing to set a dose. Optionally, the set dose can be viewed through a window in the housing as markings or numbers printed on the dose setting element. Once a dose has been set, and optionally corrected by partially returning the dose setting element towards the housing, the set dose can be expelled by actuating the dose delivery mechanism, for example using an actuator such as a delivery button on the device. Upon actuating, the dose setting element rotates and/or axially translates into the housing and the delivery mechanism is activated, for example by a clutch or clutch mechanism. The set dose is expelled by a piston rod that advances the plunger in the attached medicament containment. The piston rod is advanced either by manual operation of the device, e.g. returning the dose setting element using the patient's thumb pushing the actuator. Alternatively the piston rod is advanced by a spring that is pre-stressed or stressed during dose setting and the spring force is released upon actuation, or the piston rod is advanced using an electromotor or by the energy stored in hydraulic, pneumatic or pyrogenic power packages.

The dose setting and delivery mechanism comprises a dose limiting mechanism preventing the setting of a dose that is above the amount of medicament left in the containment. After repeated dose settings and deliveries, the containment has emptied and contains less medicament than can be set as a maximum dose with the dose setting mechanism, in this case the dose limiting mechanism prevents the setting of a dose beyond the residual amount of medicament left in the containment. This is a safety feature as the user cannot set a higher dose then the amount that is still available, which in the absence of such a limiting mechanism could lead to under dosing. Such dose limiting mechanisms comprise a dose limiter which is either directly connected or linked to the piston rod of the dose delivery mechanism, for example a nut that is threadedly engaged with the piston rod (for example in WO0195959), or the dose limiter is linked to another part, for example threadedly, splined or rotationally engaged with the clutch and relative axial and/or rotational movement between the dose setting element and the clutch advances the limiter towards a stop. Once the limiter engages the stop, further dose setting is prevented. The dose limiting mechanism is configured to limit the dose setting to a predefined number of units of medicament that can be expelled. The predefined units is the sum of the dispensable units corresponding to the volume of medicament present in the containment and a number of priming units that the user can set and expel to advance the piston rod and close a gap between the piston rod and the plunger (as will be explained below) and/or advance the plunger to expel residual air present in the containment before setting and injecting the desired dose.

The dose setting and delivery mechanism is assembled with the medicament containment using the containment holder to form the injection or infusion device. Due to the dimensional tolerances of the containment, and the position of the plunger in the containment, variations of the containment holder and the housing of the dose setting mechanism, there may be substantial play between the end of the piston rod and the proximal end of the plunger after assembly of the injection or infusion device. The user must therefore set and expel a number of priming units to close the gap and ensure that the piston rod abuts the plunger for advancement. The gap that needs to be overcome varies from pen to pen and the user will have to set a different number of priming units before the pen is ready for injection. Alternatively, there is no gap between the end of the piston rod and the plunger and upon assembly, for example when the point of no return for an irreversible snap-fit connection is reached and the piston rod will advance the plunger, thereby pressurizing the liquid in the containment which immediately expels upon attachment of a needle to the injection device.

There is a need to provide an assembly method where a user experiences virtual identical priming conditions across individual pen samples, e.g. with a constant and reduced clearance between the piston rod and the plunger of the containment independent from dimensional tolerances that arise from the parts (subassemblies) of the injection or infusion device or the plunger position in the cartridge. The gap can may be minimized or even closed (no priming) during assembly of the device.

In WO2017001694, the clearance between the piston rod and the plunger is reduced during device assembly. The dose setting and expelling mechanism comprises a dose limiting mechanism and it is disclosed that the dose limiting mechanism is always at the initial position (e.g. capable of counting and accumulating the sum of dispensable doses and the total amount of priming doses) independent from the specific assembly with a cartridge having different axial positions for the plunger. The dose setting mechanism does not comprise the piston rod but the piston rod is a separate part that is inserted into the cartridge holder using a nut after insertion of the cartridge into the holder but prior to assembly with the dose setting mechanism. The piston rod position with respect to the plunger is adjusted to reduce or minimize the clearance between the piston rod and the plunger. The pre-assembly of the cartridge, cartridge holder, piston rod and nut is subsequently joined with the dose setting mechanism. As the dose setting mechanism is not used during assembly, the dose limiting mechanism remains at the initial position. A disadvantage of this assembly method is that for cartridges where the plunger position requires a substantial amount of piston rod advancement for gap clearance, is that the during use the piston rod will advance to the end of the cartridge, and cannot advance furthermore but the dose limiting mechanism is not active yet. Thus for the last dose the user can expel from the cartridge, the limiting mechanism will not be activated which is a potential health risk due to under dosing.

It is an object of the present invention to provide an assembly method including factory priming that reduces or eliminates the gap between the piston rod and the plunger that ensures that the dose limiting mechanism is capable of limiting the last dose independent from the initial gap present. This objective is achieved by the assembly method of claim 1.
It is a further object to provide consistent gaps between the piston rod and the plunger between different pens, e.g. the user experiences a constant number of user priming doses to increase the reliability of the device after assembly. This objective is achieved by the assembly method of claims 2 and 3.

Another object is to provide a method step that ensures that outliers in plunger position, either with no or a very small initial clearance or a very high clearance that would exceed the number of priming units programmed in the limiting mechanism will be discarded from the assembly method. These objectives are achieved by the assembly method presented in claims 6 to 10

### Definitions

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from - or harvested by - biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or direction is towards the dispensing end, the proximal end is opposite to the distal end.

### Presentation of the Invention

A method for assembly of an injection or infusion device is presented which comprises the following steps:
i) providing a first housing part including a cartridge comprising a barrel filled with a medicament and closed by a plunger. The first housing part is preferably a cartridge holder and before step i) the filled cartridge is inserted into the cartridge holder (or first housing part). The filled cartridge comprises a septum that can be pierced by a needle and the liquid medicament is enclosed by the barrel between the septum and the plunger.
ii) providing a second housing part configured to be connected with the first housing part, comprising:
   a. a drive mechanism configured to advance a piston rod for advancing the plunger in a distal direction in the cartridge,
   b. a dose setting mechanism for setting dose units to be expelled from the injection or infusion device. The dose setting mechanism comprises a dose setting element such as a dose sleeve that can be moved, preferably rotated, with respect to the second housing part for setting a dose,
   c. a dose limiting mechanism configured to count and accumulate the set dose units and limit the dose setting to a sum of dispensable units and priming units. The dose limiting mechanism counts each dose set and accumulates the set and expelled doses, e.g. when a new dose is set the limiting mechanism adds this set value to the previous set and expelled doses.
iii) measuring the position of a proximal end of the plunger with respect to the first housing part and the position of the distal end of the piston rod with respect to the second housing part.
iv) calculating a distance (d) between the distal end of the piston rod and the proximal end of the plunger if the first and second housing parts were connected with each other. From the relative position of the plunger with respect to the first housing part and the relative position of the piston rod with respect to the second housing part, the distance between the plunger and the piston rod in an assembled state can be calculated once the first and second housing parts are connected as the position of the connectors between the first and second housing parts is known.
v) select a dose and advance the piston rod using the dose setting and drive mechanism if the calculated distance (d) is between a minimum distance (dₘᵢₙ) and a maximum distance (dmax), thereby operating the dose limiting mechanism.
vi) connecting the first housing part comprising the cartridge and the second housing part to form the injection or infusion device.

During step v) the dose limiting mechanism is operative, having the effect that also the priming steps are counted during the method (factory priming) and deducted from the total sum of dispensable and priming doses, having the advantage that it is prevented that the limiting mechanism is not active yet while the plunger has advanced to the end of the cartridge. Assuming that a plunger is positioned relatively close to the septum and the counter of the limiting mechanism is still capable of counting the total sum of doses, then the counter is not at zero when the plunger has reached the end of the cartridge thereby not preventing or limiting the last dose as the limiter is not working. The last dose is not limited in this case during dose setting but during dose delivery, which reduces the safety and usability of the device. Furthermore, pens with a calculated distance below the minimum distance dₘᵢₙ and above the maximum distance dₘₐₓ are identified. For the distance being above dₘₐₓ this implies identifying the combination of first and second housing parts with a cartridge that potentially suffer from a non-working end of content mechanism. For the distance being below the minimum distance dₘᵢₙ identifying those combinations between first and second housing parts with a cartridge with a potential for abutment between the piston rod of the second housing part and the plunger in the cartridge present in the first housing part after assembly. These potential outliers are identified prior to assembly step vi) meaning that a) improving the reliability and safety of the final product and b) cartridges with (expensive) medicament can be reworked in the manufacturing process such that they are within specifications after rework. Or the outliers are paired in a separate manufacturing step. Thereby the number of out of specification devices after final assembly is reduced.

The assembly method wherein in (or during) step (v) the dose is selected (in the factory) and subsequently the piston rod is advanced according to that selected dose to ensure a fixed distance (d_{fix}) between the piston rod and the plunger after connecting step (vi). The fixed distance d_{fix} ensures that the number of priming units the user needs to dial is reduced, and ideally always the same, e.g. for all pens the number of "empty" clicks before medication is expelled is the same. This increases the reliability of the device and prevents loss of medicament due to priming by the user.

The assembly method wherein the fixed distance (d_{fix}) corresponds to the distance of one unit that the piston rod can be advanced by the drive mechanism. This ensures that the user needs to set only one unit, e.g. the minimum unit that can be set with the dose setting device for the user priming step. The fixed distance d_{fix} preferably corresponds to the distance that the piston rod travels if one dose unit is set and expelled.

In the assembly method the dose limiting mechanism comprises a dose limiter which is moved from an initial position corresponding to the sum of dispensable units and priming units to a second position corresponding to the advanced piston rod during factory priming step (v). The dose limiter moves from an initial position that refers to a full cartridge towards a stop and once the limiter abuts or engages the stop, further dose setting by the dose setting mechanism is prevented by the dose limiting mechanism. When the dose limiter abuts the stop, then this position corresponds to an empty cartridge and during repeated dose settings and dose deliveries the limiter has travelled from the initial position to the stop position. The pathway that the limiter travels may be a pure rotational, a pure axial or a combination between an axial and rotational pathway, for example a helical pathway.

The dose limiter of the limiting mechanism is preferably arranged between a dose setting element of the dose setting mechanism and the drive mechanism, preferably between a dose sleeve and a driver or clutch of the drive mechanism. For example a nut element is threadedly engaged with a driver or clutch and splined to the dosing sleeve. During dose setting, the dosing sleeve is rotated versus a non-rotating driver or clutch and the nut travels along the clutch or driver as the nut follows the rotation of the dose sleeve. At the end of the threaded engagement between the nut and the driver or clutch, there is an axial or radial stop preventing further rotation of the nut element and therewith preventing rotation of the dose sleeve, thus limiting the dose setting. During dose delivery, there is no relative rotation between the driver (or clutch) and the dose sleeve and the position of the nut is frozen until the next dose will be set. As another alternative, there is a stop wheel positioned between the dose sleeve and the clutch or driver. The stop wheel is engaged with the clutch and received by bearings that allow rotation of the stop wheel with respect to the clutch. The stop wheel has a toothing on the outside engaging a toothing on the inside of the dose sleeve such that relative rotation between the non-rotating clutch (during dose setting) and the rotating dose sleeve rotates the stop wheel from an initial position towards a stop position. During dose delivery there is no relative rotation between the clutch and the dose sleeve therefore the relative position of the stop wheel with respect to the clutch and the dose sleeve is fixed. After a defined number of revolutions, e.g. dosing steps, the stop wheel is tilted by additional stop means present on the stop wheel and dosing sleeve and therewith directly or indirectly blocking further rotation and dose setting. The advantage of the stop wheel is that there is, in contrast to the nut member mentioned before, no axial movement thereby reducing the amount of axial space needed for the dose limiting mechanism. Alternatively the dose limiter is arranged between the piston rod and the dose setting element, preferably the dose limiter is directly engaged with a threaded piston rod. For example a nut engages the piston rod and during dose setting, the nut is directly or indirectly engaged with the dose sleeve and travels along the threads of the piston rod towards a stop. During dose delivery, the relative position between the limiter and the piston rod is fixed. In the stop position, the limiter abuts the stop and prevents further dose setting. For all alternatives, the clutch, the driver and the piston are prevented from rotational movement and/or axial movement by a backdrive safety, for example by a one-way ratchet mechanism present between the clutch, the driver or the piston rod and the housing. The one-way ratchet mechanism may comprise an axial and/or a radial ratchet member.

In the assembly method the first housing part comprising the cartridge may be discarded, for example taken from the assembly line during in-line testing (or in process control, IPC) and connecting step (vi) of the first and second housing parts is not executed if the calculated distance (d) during step (iv) is below the minimum distance (dmin). Preferably, the minimum distance (dₘᵢₙ) corresponds to one unit that the piston rod can be advanced by the drive mechanism, e.g. the distance advanced by the piston rod if one unit is set and expelled. If the calculated distance is below the minimum distance (dmin). then there is the risk that the distal end of the piston rod abuts the proximal end of the plunger in the cartridge as the connecting or engagement means between the first and second housing part are fixed, e.g. when the "point of no return" for the assembly of the first and second housing part is reached (step vi), then the piston rod could advance the plunger or pressurize the medicament. If a user attaches a needle on the cartridge holder and pierces the septum of the cartridge, then immediately (and uncontrolled) medicament will be ejected which is not desired. Therefore, this combination of first and second housing parts with the cartridge is discarded from the assembly method to increase the reliability of the product obtained by the method. The drive assembly advances the piston rod in discrete units and the minimum unit is one unit, factory priming with a piston rod advancement below one unit cannot be accomplished.

In an alternative, the combination of first and second housing parts is not discarded from the assembly line even if there exists the risk of abutment between the end of the piston rod and the plunger in the cartridge, a fixed and controlled "minus" or overlap gap, may be allowed as the plunger in the cartridge and the dosing mechanism have a certain degree of reluctance or elasticity that prevents a dose from being expelled once a needle is attached by the user.

The assembly method wherein the first housing part comprising the cartridge is discarded, for example taken out of the assembly line during IPC, and connecting step (vi) is not executed if the calculated distance (d) is above the maximum distance (dmax). Preferably, the maximum distance (dₘₐₓ) corresponds to the number of priming units that the piston rod can be advanced by the drive mechanism. The number of priming units ranges between 1 and 25 units, preferably between 2 and 20 units, more preferably between 5 and 15 units, more preferably equals 10 units. The effect is that if the calculated distance (d) is above the maximum distance (dmax), then there is a large gap between the distal end of the piston rod and the plunger after assembly. This would require the setting and dispensing of many "empty" priming doses during factory priming before the plunger in the cartridge has advanced enough. If the calculated distance is above the number of priming units that may be counted by the limiting mechanism, for example the calculated distance would correspond to 18 units whereas the sum of priming units (for example 10) and dispensable units (300) amounts 310 units, then factory priming the device with the 18 "empty" units will move the dose limiting mechanism to a position after priming to 310-18 = 292 Units. If the user sets and dispenses multiple doses this will result in that the stop mechanism holds after a distance corresponding to 292 units which may be below the 300 units that are claimed to be available to the user. This could result in a "label claim" by the user as less units can be expelled then expected by the user because the piston rod is halted by the limiting mechanism.

The first housing part defines a first longitudinal axis and the second housing part defines a second longitudinal axis. The first and second housing parts are preferably shaped as a cylinder and the first and second longitudinal axis are the central axis of the cylinders. Alternatively, the first and second housing parts are shaped as half open boxes with a rectangular base and the longitudinal axis goes through the center of the base. During assembly, particularly between steps ii) and iii) of the method, the first and second longitudinal axis are aligned with each other, or are at least parallel to another and the first and second housing parts are preferably spaced apart from each other. This has the advantage that measuring equipment, for example a tactile measuring machine can enter the space and measure the position of the plunger in the cartridge and the position of the first housing part whereas the position of the second housing part and the piston rod can be measured independently from another. Preferably the first and second housing parts are arranged in a measuring fixture that is preferably part of an assembly line. The measuring step (iii) is optionally part of the IPC check. The measuring fixtures may have reference marks or edges that are used to measure the relative position between the housing part and the fixture and therewith ensuring also accurate measurement of the position of the cartridge and plunger with respect to the first housing part or the position of the piston rod with respect to the second housing part. The positions can be measured, for example, using a tactile measuring machine, an optical measuring machine (for example using a laser, or a CCD camera) or a measuring gauge.

The first and second housing parts are mutually engaged to form an irreversible connection after step (vi), and this can be done using an irreversible connector for a disposable device and using a reversible connector for a reusable device. The irreversible connection is preferably a snap fit connection using connectors comprising flexible arms, protrusions and cut outs that are part of the first and second housing parts. Ramped surfaces may be part of the connectors and stop limiters, for example a circumferential protrusion, may arrest the first and second housing parts once they passed the point of "no return" for the snap fit connection. Such circumferential protrusions may be used for the tactile or optical measuring machine to accurately define the position of the first and/or second housing parts. The connectors for establishing the irreversible connection may be configured for a sole axial approach of the first and second housing parts but may also be configured to be combined with a relative rotational movement between the first and second housing parts. The reversible connection between the first and second housing parts may be a screw type of connection or a bayonet type of connection.

The dose limiting mechanism performs a countdown of dispensed units during repeated dose setting and delivery, starting from the sum of the dispensable units and the priming units. The number of dispensable units preferably corresponds to the dispensable volume of the medicament present in the cartridge. For example a cartridge may be filled with liquid corresponding to 300 units of the medicament, and the number of dispensable units counted by the dose limiting mechanism thus corresponds to that amount of 300 units. The dose limiting mechanism does not account for the so called "dead volume" in the neck of the cartridge which correspond to the non-dispensable units.

The piston rod may comprise a flange attached to or integrally formed with the distal end of the piston rod. There may be a bearing, for example a pivot bearing formed by a ball and socket, between the flange and the piston rod allowing for relative rotation therebetween. The piston rod may also comprise an additional optical marking or a dedicated landmark for the optical respectively tactile measurements. The plunger in the cartridge may also comprise means to facilitate the optical or tactile measurements, for example a colored or reflective marker that can be identified by a camera.

Furthermore, an injection or infusion device is disclosed that is assembled using the assembly method described above. The injection device may be a conventional pen for setting variable doses with a dosing sleeve that is rotated and translated out of the second housing for dose setting and returned manually during dose delivery. Alternatively it may be a fixed dose pen with a pull-push dose setting knob that is manually returned without rotation during dose delivery. Alternatively it may be an autopen where a dose is set manually and dispensed by a delivery mechanism driven by an electromotor, alternatively the injection device may be a spring driven device where a dose is set by rotating a dose setting knob and the dose is delivered by releasing the energy stored in a spring. Alternatively it may be a spring driven autoinjector where a prefilled syringe is emptied during dose delivery.

Furthermore an infusion device is disclosed that is assembled according to the method described above, the infusion device may be an infusion pump, a bolus injector that may be attached to the skin of the patient or it may be a patch injector.

### Detailed description of the drawings

Various embodiments will be explained below with reference to the figures. A person skilled in the art will accordingly recognize that various changes and modifications can be made to the embodiments displayed below without deviating from the invention or leaving its scope of protection.

List of the drawings:
Figure 1: Prior art injection pen with a dose limiting mechanism comprising a stop wheel as a dose limiter (non-axial working end of content mechanism).
Figure 2: Alternative drive sleeve or clutch taken from the prior art with a nut as a dose limiter (axial working end of content mechanism).
Figure 3: Cartridge as an example of a medicament containment.
Figure 4: Cartridge as an example of a medicament containment inserted in a cartridge holder (first housing part).
Figure 5: Distal end of a dosing and delivery mechanism embodied in a second housing part.
Figure 6: Detail of an assembled first and second housing part showing the clearance or gap between the proximal end of the plunger in the cartridge and the distal end of the piston rod (flange).

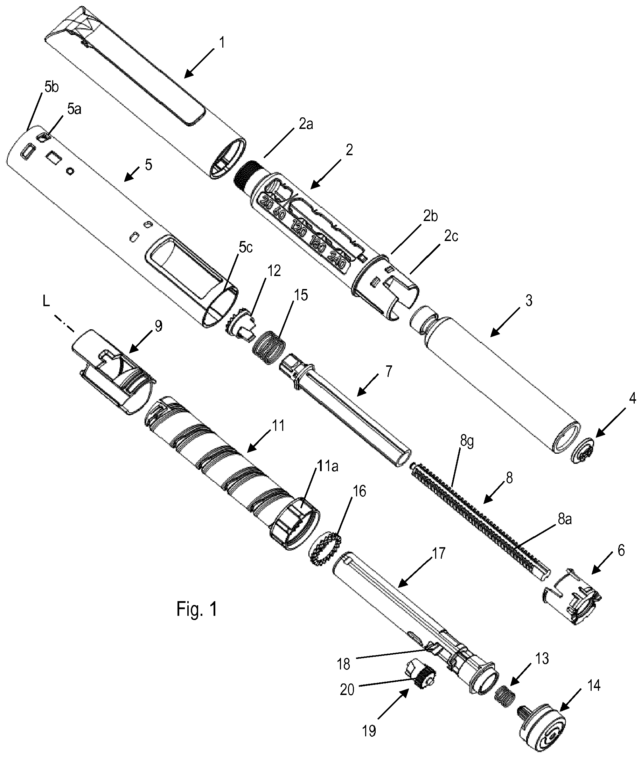

Figure 1 shows an exploded view of the individual parts of a prior art injection device as disclosed in WO2013170392 and having a non-axial working dose limiting mechanism comprising a stop wheel, the parts and functioning will be described hereafter.

Dose setting: A dose dial sleeve (11) is threadedly engaged with a housing (5) or with an insert (9) that may be part of the housing, such that rotation of the dose dial sleeve (11) by rotating dose dial grip (11a) displaces the dose dial sleeve (11) out of the housing (5). A dose can be corrected by rotating the dose dial sleeve (11) in the opposite direction and it will return the dose dial sleeve (11) back towards the proximal end (5c) of the housing (5).

Housing (5) is shaped as a tubular element having a proximal end or rim (5c) and a distal end or rim (5b). A cartridge (3) comprising a not shown medicament and closed by a not shown plunger, can be inserted in cartridge holder (2). The cartridge holder comprises an extension (2d) and an arrester rim (2c). The extension (2d) of the cartridge holder (2) can be inserted into the distal end (5b) of the housing (2) until protrusions (2b) of the cartridge holder snap fit into cut outs (5a) of the housing (5) and the arrester ring (2c) abuts the distal rim (5b) of the housing to form an irreversible connection or a connection that would require plastic deformation of the protrusions (2b) and/ or cut outs (5a) to release the connection. This is a typical example for a connection for a disposable injection pen. The cartridge holder (2) comprises a threading (2a) at the outside surface of the distal end and is adapted for engaging of a not shown needle. The cartridge holder can be closed using a pen cap (1). The housing (5) comprises an inwardly directed flange that is not shown, having not shown protrusions engaging keyways (8a) of a piston rod (8), thereby preventing rotation of the piston rod (8) with respect to the housing (5) whereas allowing for axial movement of the piston rod. The piston rod (8) comprises a threading (8g) on the outside engaging a threading or thread segment on the inside of a drive nut (7). Thus rotation of the drive nut (7) in one rotation direction results in a (desired) piston rod advancement for advancing the plunger in the cartridge (3) and expel medication. Rotating the drive nut (7) in the opposite rotation direction would result in an (unwanted) piston rod retraction into the device. To prevent the piston rod (8) form retracting, a backdrive safety mechanism (6, 7, 15, 12, 5) is part of the injection device. A toothed wheel (12) is rotationally locked to the drive nut (7) whereas the toothed wheel (12) can axially move against the bias of a spring (15) with respect to the nut (7). The toothed wheel (13) has asymmetric saw teeth arranged on the circumference and pointing into the distal direction that engage matching saw teeth present on the not shown flange in the housing (5). The asymmetric saw teeth arrangement on the housing (5) and the toothed wheel (12) form a one-way ratchet that is biased by spring (15) and the assembly is engaged with the housing (5) using insert (6). The one-way ratchet ensures that the toothed wheel (13), and due to its rotational locked engagement also the drive nut (7), can only rotate in the rotation direction for advancing the piston rod (8). The opposite rotation direction driving the piston rod (8) back into the housing (5) is blocked. Rotation of the toothed wheel (13) in the direction for piston rod advancement requires a certain reluctance that needs to be overcome during dose delivery and this reluctance is sufficient to prevent rotation of the toothed wheel (12) and threaded nut (7) during dose setting.

Coupling mechanism: A coupling sleeve (17) is positioned coaxially within the dose dial sleeve (11). The coupling sleeve (17) is splined to the drive nut (7), e.g. the coupling sleeve (17) can axially move with respect to the drive nut (7) but both parts are rotationally locked to each other. The coupling mechanism comprises a click ring (16) that is positioned between a radially extending flange on the coupling sleeve (17) and the dose dial grip (11a). The click ring (16) has two sets of asymmetric saw teeth that are disposed on the circumference and one set of saw teeth point axially in the distal direction and which are configured for engaging not shown saw teeth on the inside of the dose dial grip (11a). A second set of click ring saw teeth point axially in the proximal direction and are configured to engage asymmetric saw teeth present on the flange of the coupling sleeve (17). The coupling mechanism is biased by a spring (13) that is sandwiched between the dose dial grip (11) and actuation button (14) such that the saw teeth of the coupling sleeve's flange, the click ring (16) and the dose dial grip (11a) are in engagement.

Dose limiting mechanism: A stop wheel (19) is inserted in a cut out (18) of the coupling sleeve (17). The stop wheel (19) has a central axis that is received in bearings present in the cut out (18) such that the stop wheel (19) can rotate, but not axially move with respect to the coupling sleeve (17). The stop wheel (19) has a toothing (20) on the outside that is arranged parallel to longitudinal axis (L) of the housing (5) and the toothing (20) matches a not shown complementary toothing on the inside of the dose dial sleeve (11). During dose setting, the user grasps the dose dial grip (11a) to rotate the dose dial sleeve (11) with respect to the housing (5). Due to the threaded engagement between the dose dial sleeve (11) and the housing (5) or housing insert (9) the dose dial sleeve axially displaces with respect to the housing and simultaneously slaves the clutch mechanism with the click ring (16) and the coupling sleeve (17) in the proximal direction. The set dose can be viewed through a window in the housing (5) as numbers printed onto the dose dial sleeve (11). Minimum and maximum dose stops are present between the dose dial sleeve (11) and the housing (5) or housing insert (9), preferably as a radial stops. A dose is corrected by rotation of the dose dial sleeve (11) in the opposite direction. During dose setting and dose correction, the backdrive safety mechanism including the one-way ratchet prevents the nut (7) from rotating and therewith the coupling sleeve (17) that is splined to the nut (7). Due to the relative rotation between the dose dial sleeve (11) and the non-rotating coupling sleeve (17), the click ring (16) with its saw teeth is able to provide the dose setting and dose correction clicks corresponding to the set dose units.

During dose delivery, the user presses the actuation button (14) with his thumb after having set the desired dose and the axial pressure closes the clutch mechanism between the coupling sleeve (17), the click ring (16) and the dose dial sleeve (11) thus forcing the coupling sleeve (17) and click ring (16) back into the housing. The dose dial sleeve (11) is threadedly engaged with the housing (1) or insert (9) and rotates back into the housing and this rotation is transmitted to the coupling sleeve (17) and the nut (7) thereby advancing the piston rod (8) and the plunger in the cartridge for expelling the set dose. During dose delivery, the dose dial sleeve (11), click ring (16), coupling sleeve (17), drive nut (7) and toothed wheel (12) rotate together. The rotation of the toothed wheel (12) ensures that the ratchet mechanism between the housing (5) and the wheel produces the dose delivery clicks.

Dose limiting mechanism: During dose setting, there is a relative rotation between the dose dial sleeve (11) and the coupling sleeve (17) and therefore the stop wheel (19) is forced to rotate from an initial position towards a position corresponding to the set dose. During dose delivery there is no relative rotation between the dose dial sleeve (11) and the coupling sleeve (17) and therefore the position of the stop wheel is not changed. During repeated dose settings, the stop wheel (19) moves each time to a new position that is frozen during dose delivery until the cartridge is almost empty. During the last dose setting step, stop limiting means (not shown) present on the inside of the dose dial sleeve (11) and the stop wheel (19) tilt the stop wheel out of its normal position and therewith directly or indirectly blocks the relative rotation between the dose dial sleeve (11) and the coupling sleeve (17) in the dose increasing direction, whereas the set dose still can be corrected by rotation of the dose dial sleeve (11) in the dose decreasing direction. In this position, the torque generated by the dose dial sleeve in is transmitted to the stop wheel, the coupling sleeve and via the drive nut to the one way ratchet guided to the housing. The stop wheel (19) is present in the cut out (18) of the coupling sleeve (17) and there is no relative axial movement between the stop wheel and the coupling sleeve (17) during dose setting and dose delivery and therefore this is a so-called non-axial working dose limiting mechanism. In the first example, a non-rotating piston rod (8) is shown, since the piston rod is splined to the housing and the threading is provided on the drive nut; an inversion of the kinematics lies within the skills of the skilled person by reversing the location of the splined and threaded engagement, e.g. a threaded engagement between the housing and the piston rod and a splined engagement between the nut and the piston rod.

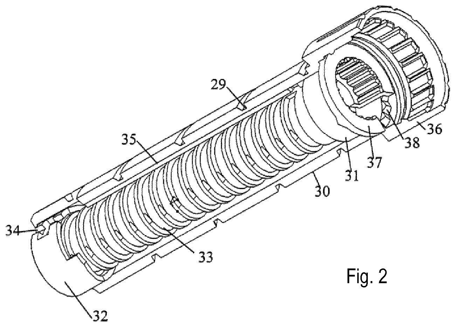

An alternative example for a prior art dose limiting mechanism that is axial working is shown in Figure 2, which is taken from WO01/19434. The coupling sleeve (31) replaces the coupling sleeve (17) of the first example whereas the dose dial sleeve (30) with its external threading (29) is identical to the dose dial sleeve (11) of the first prior art example. The coupling sleeve (31) lacks the cut-out (18) and stop wheel (19) of the first example but instead has a nut member (32) is threadedly engaged with threading (33) that is present on the outside of the coupling sleeve (31). The nut member (32) has an axial recess or keyway (34) engaging a longitudinal protrusion or key (35) present on the inside of the dose dial sleeve (30). During dose setting, the dose dial sleeve (30) rotates with respect to the non-rotating coupling sleeve (31) and due to the key/keyway engagement, the nut member (32) will travel along the threading (33) towards a stop. During dose delivery, there is no relative rotation between the dose dial sleeve (30) and the coupling sleeve (31) and the position of the nut member (32) will not change. After repeated dose settings the nut member (32) has moved axially to the stop and once the nut member (32) abuts the stop at the end of the threading (33), then further dose setting is prevented thus limiting the dose setting to the amount of medicament left in the cartridge.

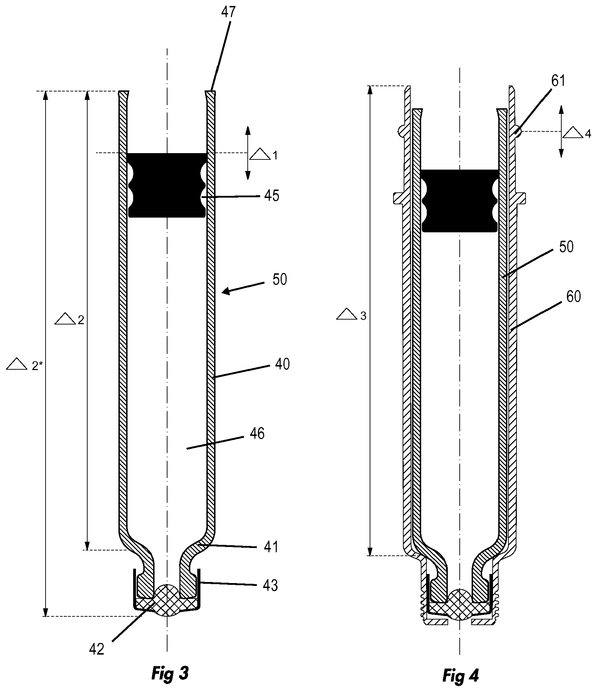

In Figures 3 to 6 the assembly method according to the present invention is explained in more detail. Figure 3 shows a cartridge (50) comprising a barrel section (40) having two openings, a pierceable septum (42) is attached to the opening on the shoulder or neck portion (41) of the cartridge (50) whereas a plunger (45) encloses medicament (46) within the barrel section. There is a relative large variation in the plunger position within the barrel, especially when first the septum (42) is attached to the shoulder using a crimp (43), followed by filling the opening at the proximal end (47) with medicament and finally closing the cartridge (50) with plunger (45) using a positioning tube. The positioning tube has a diameter below the internal diameter of the barrel (40) such that the positioning tube can be inserted into the proximal opening (47) of the cartridge with the end of the positioning tube at the desired position. The plunger (45) is subsequently compressed and pressed through the tube. Once leaving the positioning tube the plunger (45) expands and press-fits with the wall of the cartridge. At the manufacturing speeds required, there is a relatively large variation (Δ1) for the plunger position which can be in the order of magnitude of millimeters. The second source of dimensional variation is the glass barrel of the cartridge itself, the glass production inherently leads to relative large dimensional tolerances of the length (Δ2*) of the cartridge or the position (Δ2) of the neck portion or shoulder portion (41). Finally the attachment of the septum (42) with the crimp (43) results in an additional source of dimensional variation of the length as the septum, crimp and neck portion also add to the variation in length of cartridge (50).

In Figure 4, the cartridge (50) has been inserted into cartridge holder (60). The cartridge holder (60) may also be designated as the first housing part and is made of a plastic material using injection molding leading to (relatively narrow) dimensional tolerances. The stresses present in the plastic material directly after injection molding may relax over time leading to dimensional changes during shelf life ageing. Additionally the material itself may age leading to shrinkage over time. In the sum there are several sources for dimensional tolerances after insertion of the cartridge (50) into the cartridge holder (60): The length of the cartridge holder or the position of the shoulder support for the cartridge results in a dimensional tolerance Δ3 and the position of a connector (61) for connecting the first housing to the second housing is also subjected to dimensional variation Δ4.

In the assembly method, the position of the plunger with respect to the first housing part or cartridge holder is measured using tactile means (for example a 3D coordinate measuring machine), optical means (for example using a camera), or a measuring gauge to measure the position of the plunger which includes the sum of all the tolerance fields (Δ1, Δ2, A3, Δ4). Preferably, the assembly of the cartridge (50) and the cartridge holder (60) is inserted into a measuring fixture to accurately measure the position of the plunger with respect to a reference present on the cartridge holder (for example connector 61) or with respect to an external reference that is not part of the assembly.

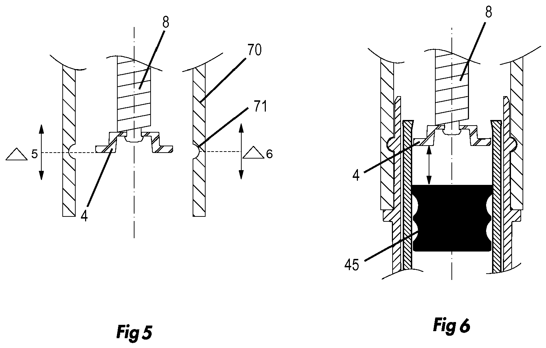

In Figure 5, a distal section of the dose setting and dose delivery mechanism is shown with housing (70), which may be the second housing part, a piston rod (8) optionally having flange (4). The housing (70) further comprises a connector (71) configured for connecting with the connector of the first housing part. Also for the positions of the piston rod (8) or flange (4) there may be a dimensional tolerance Δ5 next to a dimensional tolerance Δ6 for the position of the connector (71).

The position of the flange (4) or piston rod (8) is measured with respect to a reference which may be a reference on the housing (70), for example the connector (71) or the distal end or a circumferential rim on the housing, or alternatively, the position of the piston rod is measured with respect to an external reference. Alike the cartridge and cartridge holder, the dose setting mechanism is preferably fixated in a fixture for measuring the positions.

Once the positions of the plunger and the piston rod are known then, for each combination that is envisaged to be assembled in a final assembly step to marry the first and second housing parts into a releasable or non-releasable connection, the theoretical gap (d) is calculated between the plunger and the piston rod if the two parts would have been joined together. The theoretical gap (d) is calculated using the data from the measurement step. Depending on the calculated gap (d), the combination:
- is joined together if the gap (d) is between a minimum (dₘᵢₙ) and maximum value (dₘₐₓ) for the gap clearance, but after having set and expelled a number of doses from the dose setting mechanism prior to assembly to ensure that a fixed gap (d_{fix}) will exist after joining the two parts. During the dose setting and expelling, the dose limiting mechanism is active and counts the factory primed doses.
- may be joined together if the gap is below a minimum distance (dmin). Either the specific combination will not be joined together as there is a high risk for plunger advancement and pressurization of the incompressible liquid, or if there is only a minor abutment potential between the piston rod and the plunger (for example less than 0.5mm, preferably less than 0.2mm after joining), then the combination may be joined together
- is not joined together if the gap is above a maximum distance (dmax), to avoid a label claim.

As the specific positions for the plunger and piston rod are known, the method may foresee to select specific combinations of first and second housing parts to reduce the number of outliers and out of specification combinations and increase the efficiency of the assembly process.

A detail of an assembled injection device is presented in Figure 6 with a fixed and controlled clearance or gap (d_{fix}) between the plunger and the piston rod (or flange).

### List of reference numbers

| | | | |
|---|---|---|---|
| 1 | Pen cap | 18 | Cut out |
| 2 | Cartridge holder, first housing part | 19 | Stop wheel |
| 2a | Threading | 20 | Toothing |
| 2b | Protrusion | 29 | Threading |
| 2c | Arrester ring | 30 | Dose dial sleeve |
| 2d | Extension | 31 | Coupling sleeve |
| 3 | Cartridge | 32 | Nut member |
| 4 | Flange | 33 | Threading to nut member |
| 5 | Housing, second housing part | 34 | Recess, keyway |
| 5a | Cut out, aperture | 35 | Longitudinal protrusion, key |
| 5b | Distal end | 36 | Dose dial grip |
| 5c | Proximal rim, end | 37 | Flange |
| 6 | Insert | 38 | Toothing |
| 7 | Drive nut | 40 | Cartridge barrel |
| 8 | Piston rod | 41 | Shoulder |
| 8a | Keyways | 42 | Septum |
| 8g | Threading | 43 | Crimp |
| 9 | Insert | 45 | Plunger |
| 11 | Dose dial sleeve | 46 | Medicament |
| 11a | Dose dial grip | 47 | Proximal end |
| 12 | Toothed wheel | 50 | Cartridge |
| 13 | Spring | 60 | Cartridge holder, first housing part |
| 14 | Actuation button | 61 | Connector |
| 15 | Spring | 70 | Housing, second housing part |
| 16 | Click ring | 71 | Connector, cut out, aperture |
| 17 | Coupling sleeve | L | Longitudinal axis |

## Claims

1. A method for assembly of an injection or infusion device comprising the steps of:
i) providing a first housing part including a cartridge comprising a barrel filled with a medicament and closed by a plunger
ii) providing a second housing part configured to be connected with the first housing part, comprising:
a. a drive mechanism configured to advance a piston rod for advancing the plunger in a distal direction in the cartridge
b. a dose setting mechanism for setting dose units to be expelled from the injection or infusion device
c. a dose limiting mechanism configured to count and accumulate the set dose units and limit the dose setting to a sum of dispensable units and priming units
iii) measuring the position of a proximal end of the plunger with respect to the first housing part and the position of the distal end of the piston rod with respect to the second housing part
iv) calculating a distance (d) between the distal end of the piston rod and the proximal end of the plunger if the first and second housing parts were connected with each other
v) select a dose and advance the piston rod using the dose setting and drive mechanism if the calculated distance (d) is between a minimum distance (dₘᵢₙ) and a maximum distance (dmax), thereby operating the dose limiting mechanism
vi) connecting the first housing part comprising the cartridge and the second housing part to form the injection or infusion device,

2. The method according to claim 1 wherein in step (v) the dose is selected to ensure a fixed distance (d_{fix}) between the piston rod and the plug after step (vi),

3. The method according to claim 2 wherein (d_{fix}) corresponds to the distance to expel one dose unit that the piston rod can be advanced by the drive mechanism,

4. The method according to any of the previous claims wherein the dose limiting mechanism comprises a dose limiter and wherein the dose limiter is moved from an initial position corresponding to the sum of dispensable units and priming units to a second position corresponding to the advanced piston rod during step (v),

5. The method according to claim 4 wherein the dose limiter is arranged between a dose setting element of the dose setting mechanism and the drive mechanism, preferably a driver of the drive mechanism, or the dose limiter is arranged between the piston rod and the dose setting element, preferably the dose limiter is directly engaged with the piston rod,

6. The method according to any of the previous claims wherein the first housing part comprising the cartridge is discarded and step (vi) is not executed if the calculated distance (d) is below the minimum distance (dmin),

7. The method according to any of the previous claims wherein the minimum distance (dₘᵢₙ) corresponds to the distance to expel one dose unit that the piston rod can be advanced by the drive mechanism,

8. The method according to any of the previous claims wherein the first housing part comprising the cartridge is discarded and step (vi) is not executed if the calculated distance (d) is above the maximum distance (dmax),

9. The method according to any of the previous claims wherein the maximum distance (dₘₐₓ) corresponds to the number of priming units that the piston rod can be advanced by the drive mechanism,

10. The method according to any the previous claims wherein the number of priming units ranges between 1 and 25 units, preferably between 2 and 20 units, more preferably between 5 and 15 units, more preferably equals 10 units,

11. The method according to any of the previous claims wherein the first housing part defines a first longitudinal axis and the second housing part defines a second longitudinal axis and wherein between steps ii) and iii) the first and second longitudinal axis are aligned with each other and the first and second housing parts are spaced apart from each other, preferably the first and second housing parts are arranged in a measuring fixture,

12. The method according to any of the previous claims wherein the first and second housing parts are mutually engaged to form an irreversible connection after step (vi), preferably via a snap fit connection,

13. The method according to any of the previous claims wherein the position of the plunger and the position of the piston rod in step (iii) is measured using optical or tactile measuring means, preferably a laser or tactile measurement machine,

14. The method according to any of the previous claims wherein the dispensable units correspond to the dispensable volume of the medicament present in the cartridge,

15. The method according to any of the previous claims wherein the piston rod comprises a flange at the distal end.
